# EUROPÄISCHE PATENTSCHRIFT

(11) **EP 2 898 818 B1**
(45) Veröffentlichungstag und Bekanntmachung des Hinweises auf die Patenterteilung: **06.03.2019**
(21) Anmeldenummer: 15150628.4
(22) Anmeldetag: 09.01.2015
(51) Int. Cl.: A61B 1/008, A61B 17/29

(54) **Endoskopisches Instrument**
Endoscopic instrument
Instrument endoscopique

(30) Priorität: 24.01.2014 DE 102014100840
(43) Veröffentlichungstag der Anmeldung: 29.07.2015
(73) Patentinhaber: Karl Storz SE & Co. KG, 78532 Tuttlingen (DE)
(72) Erfinder: Stefan, Jochen, 88639 Wald (DE); Müller, Annika, 72351 Geislingen (DE)
(74) Vertreter: Witte, Weller & Partner Patentanwälte mbB

(56) Entgegenhaltungen:
- EP-A1- 1 915 950
- WO-A1-2013/116692
- US-A1- 2005 096 694
- US-A1- 2007 152 014

## Beschreibung

Die vorliegende Erfindung betrifft ein endoskopisches Instrument mit einem ersten Abschnitt, der eine erste Längserstreckung aufweist, sowie mit einem zweiten Abschnitt und einem dritten Abschnitt.

Bei starren endoskopischen Instrumenten ist eine Verlagerung des distalen Endes besonders einfach und intuitiv möglich. Dies ist darin begründet, dass jede Bewegung am proximalen Ende unmittelbar eine vorhersehbare Verlagerung des distalen Endes bewirkt. Auch eine Kraftübertragung vom proximalen zum distalen Ende ist unmittelbar gegeben.

Da starre endoskopische Instrumente üblicherweise durch einen natürlichen oder künstlich geschaffenen Kanal in den Körper eingeführt werden, ist die Verlagerung des distalen Endes aber nur eingeschränkt möglich. Mit zunehmender Länge des Einführkanals nimmt der mögliche Bewegungsraum des distalen Endes immer weiter ab. Daher besteht der Wunsch, das distale Ende mit größerer Freiheit verlagern zu können, selbst wenn das endoskopische Instrument insgesamt nur sehr eingeschränkt bewegt werden kann.

Es gibt daher eine Vielzahl von Lösungen für flexible Endoskope. Allerdings kann die Steuerung des distalen Endes dabei manchmal nur ungenau und zu wenig intuitiv erfolgen. Zudem fehlt es oftmals an einer direkten Rückmeldung vom distalen Ende an das proximale Bedienelement. Schließlich kann es auch sehr aufwendig sein, eine ausreichende Kraftübertragung vom proximalen Ende zum distalen Ende des endoskopischen Instruments bereitzustellen.

WO 2013/116692 A1 zeigt ein endoskopisches Instrument, bei dem mittels eines kugelförmigen Bedienelements am proximalen Ende des endoskopischen Instruments ein Aktuator am distalen Ende endoskopischen Instruments verlagert werden kann. Die Übertragung der Kraft vom Bedienelement auf den Aktuator erfolgt über Stäbe.

EP 1 915 950 A1 zeigt ein endoskopisches Instrument, bei dem mittels einer stabförmigen Handhabe am proximalen Ende des endoskopischen Instruments ein Aktuator am distalen Ende endoskopischen Instruments verlagert werden kann. Die Übertragung der Kraft von der Handhabe auf den Aktuator erfolgt über Zugseile.

Es ist daher eine Aufgabe der vorliegenden Erfindung, ein verbessertes endoskopisches Instrument aufzuzeigen, das am distalen Ende abgewinkelt werden kann, bevorzugt mehrfach abgewinkelt werden kann. Dabei soll insbesondere eine einfache Kraftübertragung vom proximalen Ende zum distalen Ende möglich sein und eine gute Kraftrückmeldung vom distalen Ende an das proximale Ende gegeben sein.

Die Aufgabe wird gelöst durch ein endoskopisches Instrument gemäß Anspruch 1.

Dieser Aufbau ermöglicht es, durch ein Abwinkeln am ersten Hauptgelenk eine Abwinkelung am zweiten Hauptgelenk zu erzielen. Die Kraftübertragung kann dabei bevorzugt rein mechanisch erfolgen, insbesondere ohne eine motorische Unterstützung.

Der Hauptdruckstab ist starr ausgebildet, so dass eine Kraft, die durch eine Abwinkelung des zweiten Abschnitts auf den Hauptdruckstab übertragen wird, direkt an das zweite Hauptgelenk übertragen wird. Es kann so eine besonders zuverlässige Funktionsweise erreicht werden.

Mittels der Anordnung des ersten Untergelenks am ersten Hauptgelenk und des zweiten Untergelenks am zweiten Hauptgelenk kann bei bevorzugten Ausgestaltungen eine Übersetzung der Abwinklung am ersten Hauptgelenk auf die Abwinklung am zweiten Hauptgelenk erzielt werden. Für ein besseres Verständnis dieses Aspekts wird eine erste Mittelachse des ersten Abschnitts zur Orientierung verwendet, die sich durch einen ersten Mittelpunkt des ersten Hauptgelenks und durch einen zweiten Mittelpunkt des zweiten Hauptgelenks erstreckt. Dabei sollen diese Mittelpunkte insbesondere jeweils als der Punkt verstanden werden, um den sich das jeweilige Hauptgelenk verschwenkt.

Wird das erste Untergelenk in einem geringeren Abstand zur ersten Mittelachse angeordnet als das zweite Untergelenk, so erzeugt eine Abwinkelung am ersten Hauptgelenk um einen ersten Winkel eine Abwinklung am zweiten Hauptgelenk um einen zweiten Winkel, der kleiner ist als der erste Winkel. Wird das erste Untergelenk in einem Abstand zur ersten Mittelachse angeordnet, der größer ist als der Abstand des zweiten Untergelenks zur ersten Mittelachse, so führt eine Abwinklung des ersten Hauptgelenks um einen ersten Winkel zu einer Abwinklung am zweiten Hauptgelenk mit einem zweiten Winkel, der größer ist als der erste Winkel.

In Abhängigkeit von einem ersten maximalen Winkelbereich, in dem sich das erste Hauptgelenk abwinkeln soll, und einem gewünschten zweiten maximalen Winkelbereich, in dem sich das zweiten Hauptgelenk abwinkeln soll, kann die gewünschte Übersetzung zwischen erstem und zweitem Winkel gewählt werden. In gleicher Weise kann auch ein erstes Drehmoment am ersten Hauptgelenk auf ein zweites Drehmoment am zweiten Hauptgelenk übersetzt werden.

Es ist bei einer bevorzugten Ausgestaltung, aber insbesondere vorgesehen, dass der Abstand des ersten Untergelenks zur ersten Mittelachse zumindest ungefähr gleich dem Abstand des zweiten Untergelenks zur ersten Mittelachse ist. Dann ist eine Auslenkung um einen ersten Winkel am ersten Hauptgelenk gleich einer Auslenkung um einen zweiten Winkel am zweiten Hauptgelenk. Dies ermöglicht eine besonders intuitive Bedienung. Dies insbesondere, weil eine Verlagerung des zweiten Abschnitts relativ zum ersten Abschnitt eine Verlagerung des dritten Abschnitts relativ zum ersten Abschnitt mit demselben Drehmoment und demselben Betrag hervorruft. Es lässt sich zudem eine besonders bevorzugte Ausgestaltung realisieren, bei der der Hauptdruckstab zumindest ungefähr parallel zur ersten Mittelachse verläuft.

Es sei darauf hingewiesen, dass bei bestimmten bevorzugten Ausgestaltungen, der Hauptdruckstab zumindest abschnittsweise nicht parallel zur ersten Mittelachse verläuft. So ist insbesondere eine Ausgestaltung vorteilhaft, bei der das erste Untergelenk unter das zweite Untergelenk zwar im gleichen Abstand zur ersten Mittelachse angeordnet sind, die radiale Position des ersten Untergelenks aber diametral der radialen Position des zweiten Untergelenks gegenüberliegt. Bei dieser Ausgestaltung bedeutet dies insbesondere, dass der Hauptdruckstab die erste Mittelachse kreuzt. Eine solche Ausgestaltung wird als vorteilhaft angesehen, wenn es gewünscht ist, dass eine Auslenkung am ersten Hauptgelenk in eine bestimmte Richtung, z.B. nach links, zu einer gleich gerichteten Bewegung am zweiten Hauptgelenk führt.

Im Vergleich dazu: Wenn der Hauptdruckstab parallel zur ersten Mittelachse geführt wird, führt eine Abwinklung des ersten Hauptgelenks in eine bestimmte Richtung zu einer entgegengesetzten Abwinklung am zweiten Hauptgelenk. Ein Kreuzen der Mittelachse kann auch mit mehreren Hauptdruckstäben erzielt werden. Es muss dann lediglich sichergestellt werden, dass die Kreuzungspunkte mit der ersten Mittelachse entlang der ersten Mittelachse verteilt sind und hinreichend voneinander beabstandet sind.

Es sei darauf hingewiesen, dass der Begriff "Druck" im Hinblick auf die vorliegende Erfindung die Bedeutung eines positiven Drucks, also "Schub", beinhalten soll, da in der Übertragung eines Schubs vom proximalen Ende zum distalen Ende des endoskopischen Instruments einer der besonderen Aspekte der vorliegenden Erfindung gesehen wird. Dadurch wird aber nicht ausgeschlossen, dass der Begriff "Druck" auch einen negativen Druck, also "Zug", beinhaltet. Vielmehr ist es so, dass die Starrheit des Hauptdruckstabs sowohl die Übertragung eines Schubs als auch eines Zugs erlaubt.

Durch eine geeignete Kopplung des Hauptdruckstabs mit dem ersten Untergelenk und dem zweiten Untergelenk kann erreicht werden, dass mittels des Hauptdruckstabs sowohl ein Schub als auch ein Zug vom ersten Untergelenk auf das zweite Untergelenk übertragen werden kann. Wird der Hauptdruckstab mit dem ersten Untergelenk und/oder dem zweiten Untergelenk so verbunden, dass entweder in Schubrichtung oder in Zugrichtung ein Freilauf besteht, kann die Kopplung auch so ausgestaltet werden, dass nur ein Schub bzw. nur ein Zug übertragen wird. Es wird jedoch als vorteilhaft angesehen, dass der Hauptdruckstab so mit dem ersten Untergelenk und dem zweiten Untergelenk gekoppelt ist, dass sowohl ein Schub als auch ein Zug vom ersten Untergelenk auf das zweite Untergelenk übertragen wird. Es ist vorteilhaft, wenn der erste Abschnitt, einen ersten Hohlraum entlang der ersten Längserstreckung aufweist,

Es wird ferner darauf hingewiesen, dass die Verwendung des Begriffs "Längserstreckung" lediglich der Einführung eines Bezugssystems dient. Dabei soll eine Längserstreckung des endoskopischen Instruments insbesondere als die Erstreckung vom proximalen Ende zum distalen Ende des endoskopischen Instruments verstanden werden. Im Ruhezustand des endoskopischen Instruments, also im nicht-ausgelenkten Zustand, werden die Längserstreckungen der einzelnen Abschnitte zum Zwecke der Orientierung als zumindest ungefähr parallel zur Längserstreckung des endoskopischen Instruments verstanden bzw. fallen zumindest ungefähr mit der Längserstreckung des endoskopischen Instruments zusammen.

Der Begriff des "Gelenks" soll im Rahmen der Beschreibung sowohl mehrteilige Gelenke, wie z.B. ein Kugelgelenk, ein Drehgelenk oder ein Drehschubgelenk, als auch einteilige Gelenke, wie z.B. Festkörpergelenke, beinhalten. Bei einem einteiligen Gelenk ist das erste Gelenkteil dann ein erster Abschnitt des Gelenks und das weitere Gelenkteil ein zweiter Abschnitt des Gelenks.

Das weitere erste Hauptgelenkteil weist ein erstes Innengelenk auf, das im Inneren des weiteren ersten Hauptgelenkteils angeordnet ist und relativ zum weiteren ersten Hauptgelenkteil verschwenkbar angeordnet ist, wobei der zweite Abschnitt eine zweite Längserstreckung und einen zweiten Hohlraum entlang der zweiten Längserstreckung aufweist.

Dies ist vorteilhaft, weil mittels des ersten Innengelenks eine weitere Betätigung übertragen werden kann, die unabhängig von einer Verlagerung des weiteren ersten Hauptgelenkteils sein kann.

Das erste Innengelenk weist ein erstes Innengelenkteil und ein weiteres erstes Innengelenkteil auf, wobei das erste Innengelenkteil zumindest als ein Abschnitt einer Gelenkpfanne ausgebildet ist und das weitere erste Innengelenkteil zumindest als ein Abschnitt einer Gelenkkugel ausgebildet ist.

Dies ermöglicht eine besonders zuverlässige und flexible Kraftübertragung. Alternativ oder zusätzlich weist das zweite Innengelenk bei einer vorteilhaften Ausgestaltung ein zweites Innengelenkteil und ein weiteres zweites Innengelenkteil auf, wobei das zweite Innengelenkteil zumindest als ein Abschnitt einer Gelenkpfanne ausgebildet ist und das weitere zweite Innengelenkteil zumindest als Abschnitt einer Gelenkkugel ausgebildet ist. Es gelten entsprechend die gleichen Ausführungen wie zum ersten Innengelenk.

Das erste Innengelenkteil ist an dem weiteren ersten Hauptgelenkteil ausgebildet.

Dies kann konstruktiv besonders vorteilhaft sein, weil das weitere erste Hauptgelenkteil nicht nur ein Teil des ersten Hauptgelenks ist, sondern gleichzeitig das erste Innengelenkteil aufweist. Dabei kann die Ausbildung des ersten Innengelenkteils am weiteren ersten Hauptgelenkteil bevorzugt durch eine entsprechende Formgebung des weiteren ersten Hauptgelenkteils erzielt werden. Das erste Innengelenkteil wird dabei bevorzugt einstückig aus einem gleichen Material oder Materialmix mit dem weiteren ersten Hauptgelenkteil ausgebildet.

Damit ist die Aufgabe vollständig gelöst.

Bei einer bevorzugten Ausgestaltung sind das erste Hauptgelenkteil und das zweite Hauptgelenkteil am ersten Abschnitt angeordnet, das weitere erste Hauptgelenkteil am zweiten Abschnitt angeordnet und das weitere zweite Hauptgelenkteil am dritten Abschnitt angeordnet.

Diese Ausgestaltung ermöglicht einen konstruktiv einfachen Aufbau.

Bei einer bevorzugten Ausgestaltung der Erfindung ist das erste Hauptgelenkteil zumindest als ein Abschnitt einer Gelenkpfanne ausgebildet und das weitere erste Hauptgelenkteil zumindest als Abschnitt einer Gelenkkugel ausgebildet.

Diese Ausgestaltung ermöglicht eine besonders große Freiheit bei der Abwinklung des zweiten Abschnitts bzw. des dritten Abschnitts in mehrere Richtungen.

Alternativ oder zusätzlich ist bei einer bevorzugten Ausgestaltung das zweite Hauptgelenkteil zumindest als ein Abschnitts einer Gelenkpfanne ausgebildet und das weitere zweite Hauptgelenkteil zumindest als Abschnitt einer Gelenkkugel ausgebildet. Es gelten entsprechend die gleichen Erläuterungen wie zum ersten Hauptgelenkteil.

Bei einer weiteren vorteilhaften Ausgestaltung weist das erste Untergelenk ein erstes Untergelenkteil und ein weiteres erstes Untergelenkteil auf, wobei das erste Untergelenkteil am weiteren ersten Hauptgelenkteil ausgebildet ist und wobei das weitere erste Untergelenkteil am Hauptdruckstab ausgebildet ist.

Diese Ausgestaltung kann konstruktiv besonders vorteilhaft sein, weil das weitere erste Hauptgelenkteil nicht nur ein Teil des ersten Hauptgelenks ist, sondern gleichzeitig das erste Untergelenkteil aufweist. Dabei kann die Ausbildung des ersten Untergelenkteils am weiteren ersten Hauptgelenkteil bevorzugt durch eine entsprechende Formgebung des weiteren ersten Hauptgelenkteils erzielt werden. Das erste Untergelenkteil wird dabei bevorzugt einstückig aus einem gleichen Material oder Materialmix mit dem weiteren ersten Hauptgelenkteil ausgebildet. In entsprechender Weise ist es vorteilhaft, wenn das weitere erste Untergelenkteil einstückig mit dem Hauptdruckstab oder einem distalen Ende des Hauptdruckstabs ausgebildet ist. Dabei ist das weitere erste Untergelenkteil bevorzugt als Gelenkkugel am Hauptdruckstab ausgebildet.

Alternativ oder zusätzlich weist das zweite Untergelenk ein zweites Untergelenkteil und ein weiteres zweites Untergelenkteil auf, wobei das zweite Untergelenkteil am weiteren zweiten Hauptgelenkteil ausgebildet ist und wobei das weitere zweite Untergelenkteil am Hauptdruckstab ausgebildet ist. Es gelten entsprechend die gleichen Erläuterungen wie zum ersten Untergelenk.

Bei einer weiteren vorteilhaften Ausgestaltung ist das erste Untergelenkteil zumindest als ein Abschnitt einer Gelenkpfanne ausgebildet und ist das weitere erste Untergelenkteil zumindest als Abschnitt einer Gelenkkugel ausgebildet.

Diese Ausgestaltung kann eine zuverlässige und platzsparende Realisierung des ersten Untergelenkteils ermöglichen.

Alternativ oder zusätzlich ist bei einer bevorzugten Ausgestaltung das zweite Untergelenkteil zumindest als ein Abschnitts einer Gelenkpfanne ausgebildet und das weitere zweite Untergelenkteil zumindest als Abschnitt einer Gelenkkugel ausgebildet. Es gelten entsprechend die gleichen Erläuterungen wie zum ersten Untergelenkteil.

Bei einer weiteren vorteilhaften Ausgestaltung sind am ersten Hauptgelenk drei erste Untergelenke angeordnet.

Diese Ausgestaltung kann eine besonders präzise Übertragung der Verschwenkung des zweiten Abschnitts auf den dritten Abschnitt bewirken. In diesem Zusammenhang ist es bevorzugt, dass auch am zweiten Hauptgelenk drei zweite Untergelenke angeordnet sind. Dabei ist bevorzugt, jeweils eines der ersten Untergelenke mit einem entsprechenden der zweiten Untergelenke verbunden, bevorzugt über zusätzliche Hauptdruckstäbe. Es ist dabei bevorzugt, dass die drei Hauptdruckstäbe parallel zueinander verlaufen, insbesondere dabei jeweils parallel zu der ersten Mittelachse. Bei anderen bevorzugten Ausgestaltungen weist jedes der Hauptgelenke genau zwei Untergelenke auf. Bei weiteren bevorzugten Ausgestaltungen weisen die Hauptgelenke jeweils vier oder mehr Untergelenke auf.

Bei einer bevorzugten Ausgestaltung sind die ersten Untergelenke in einem Abstand von zwischen 45° und 120° relativ zueinander angeordnet, und zwar bezogen auf die genannte erste Mittelachse des ersten Abschnitts, die durch einen ersten Mittelpunkt des ersten Hauptgelenks und durch einen zweiten Mittelpunkt des zweiten Hauptgelenks verläuft. Bei einer bevorzugten Ausgestaltung sind die ersten Untergelenke in einem Abstand von zumindest ungefähr 120° relativ zueinander angeordnet.

Alternativ oder zusätzlich hierzu sind drei zweite Untergelenke des zweiten Hauptgelenks in einem Abstand von zwischen 45° und 120° relativ zueinander angeordnet, und zwar bezogen auf die genannte erste Mittelachse. Es gelten entsprechend in gleicher Weise die Ausführungen zu den ersten Untergelenken.

Bei einer bevorzugten Ausgestaltung weist das weitere zweite Hauptgelenkteil ein zweites Innengelenk auf, das im Inneren des Weiteren zweiten Hauptgelenkteils angeordnet ist und relativ zum weiteren zweiten Hauptgelenkteil verschwenkbar angeordnet ist. Wenn ein erstes Innengelenk und ein zweites Innengelenk vorhanden ist, kann eine Verlagerung des ersten Innengelenks auf das zweite Innengelenk übertragen werden und umgekehrt.

Bei einer weiteren vorteilhaften Ausgestaltung weist das weitere erste Innengelenkteil ein erstes Primärgelenk mit einem ersten Primärgelenkteil und einem weiteren ersten Primärgelenkteil auf, wobei das erste Primärgelenkteil an dem weiteren ersten Innengelenkteil ausgebildet ist.

Diese Ausgestaltung kann es auf einfache Weise ermöglichen, eine Betätigung auf das erste Innengelenk zu übertragen.

Alternativ oder zusätzlich hierzu weist das weitere zweite Innengelenkteil bei einer bevorzugten Ausführungsform ein zweites Primärgelenk mit einem zweiten Primärgelenkteil und einem weiteren zweiten Primärgelenkteil auf, wobei das zweite Primärgelenkteil an dem weiteren zweiten Innengelenkteil ausgebildet ist. Es gelten entsprechend die gleichen Ausführungen wie zum weiteren ersten Innengelenkteil.

Bei einer weiteren vorteilhaften Ausgestaltung der Erfindung weist das endoskopische Instrument ferner einen starren Nebendruckstab auf, der sich entlang der ersten Längserstreckung erstreckt, wobei insbesondere das weitere erste Primärgelenkteil am Nebendruckstab ausgebildet ist.

Diese Ausgestaltung kann eine besonders zuverlässige Kraftübertragung ermöglichen.

Alternativ oder zusätzlich hierzu ist bei einer weiteren bevorzugten Ausführungsform das weitere zweite Primärgelenkteil am Nebendruckstab ausgebildet.

Bei einer weiteren vorteilhaften Ausgestaltung weist das endoskopische Instrument einen starren Seitendruckstab auf, der sich entlang der zweiten Längserstreckung erstreckt, wobei das weitere erste Innengelenkteil ein erstes Sekundärgelenk mit einem ersten Sekundärgelenkteil und einem weiteren ersten Sekundärgelenkteil aufweist, wobei das erste Sekundärgelenkteil an dem weiteren ersten Innengelenkteil ausgebildet ist und das weitere erste Sekundärgelenkteil am ersten Seitendruckstab ausgebildet ist.

Diese Ausgestaltung kann in vorteilhafter Weise ermöglichen, dass das weitere erste Innengelenkteil eine Kraft aufnimmt, die entlang des ersten Abschnitts übertragen wird, und diese Kraft mittels des ersten Seitendruckstabs in den zweiten Abschnitt hineinüberträgt.

Alternativ oder zusätzlich hierzu weist das endoskopische Instrument bei einer weiteren bevorzugten Ausführungsform einen zweiten starren Seitendruckstab auf, der sich entlang eines dritten Hohlraums entlang einer dritten Längserstreckung des dritten Abschnitts erstreckt, wobei das weitere zweite Innengelenkteil ein zweites Sekundärgelenk mit einem zweiten Sekundärgelenkteil und einem weiteren zweiten Sekundärgelenkteil aufweist, wobei das zweite Sekundärgelenkteil an dem weiteren zweiten Innengelenkteil ausgebildet ist und das weitere zweite Sekundärgelenkteil am zweiten Seitendruckstab ausgebildet ist. Es gelten entsprechend die gleichen Ausführungen wie zu der Ausgestaltung mit dem ersten starren Seitendruckstab.

Bei einer weiteren vorteilhaften Ausgestaltung weist das erste Innengelenkteil drei erste Primärgelenke und drei erste Sekundärgelenke auf.

Diese Ausgestaltung kann eine besonders präzise Übertragung einer Bewegung mittels der ersten Primärgelenke und ersten Sekundärgelenke ermöglichen. Dabei ist es bevorzugt, wenn die drei ersten Primärgelenke untereinander jeweils um einen Winkel von zumindest ungefähr 120° bezogen auf die erste Mittelachse versetzt sind und die drei ersten Sekundärgelenke untereinander jeweils um zumindest ungefähr 120° bezogen auf die erste Mittelachse zueinander versetzt sind. Damit ist dann jeweils ein erstes Primärgelenk um jeweils ungefähr 60° zu zwei ersten Sekundärgelenken benachbart, und umgekehrt, jeweils bezogen auf die erste Mittelachse.

Alternativ oder zusätzlich hierzu weist bei einer bevorzugten Ausführungsform das weitere zweite Innengelenkteil drei zweite Primärgelenke und drei zweite Sekundärgelenke auf. Es gelten entsprechend in gleicher Weise die Ausführungen zum weiteren ersten Innengelenkteil.

Bei einer weiteren vorteilhaften Ausgestaltung ist der erste Abschnitt an einer Stelle entlang der ersten Längserstreckung in einen ersten Abschnittsteil und einen zweiten Abschnittsteil geteilt und sind der erste Abschnittsteil und der zweite Abschnittsteil mit einem Verbindungselement derart verbunden, dass ein Abstand zwischen dem ersten und dem zweiten Abschnittsteil einstellbar ist.

Diese Ausgestaltung ist vorteilhaft, weil die Fertigung vereinfacht werden kann. Insbesondere kann der Abstand zwischen dem ersten Hauptgelenk und dem zweiten Hauptgelenk sehr genau justiert werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine erste Ausführungsform eines endoskopischen Instruments in perspektivischer Darstellung;
- Fig. 2: das endoskopische Instrument gemäß Fig. 1 in der Seitenansicht;
- Fig. 3: das endoskopische Instrument gemäß Fig. 1 in einer Schnittdarstellung;
- Fig. 4: eine Ausschnittsvergrößerung aus Fig. 3;
- Fig. 5: eine vereinfachte Darstellung einer Draufsicht auf eine Stirnseite des Weiteren ersten Hauptgelenkteils;
- Fig. 6: eine zweite Ausführungsform eines endoskopischen Instruments;
- Fig. 7: das endoskopische Instrument gemäß Fig. 6 in einer Seitenansicht;
- Fig. 8: das endoskopische Instrument gemäß Fig. 6 in einer Schnittansicht;
- Fig. 9: zeigt eine Ausschnittsvergrößerung aus Fig. 8;
- Fig. 10: zeigt eine vereinfachte Darstellung einer Draufsicht auf das weitere erste Innengelenkteil; und
- Fig. 11: zeigt eine weitere Ausschnittsvergrößerung aus Fig. 8.

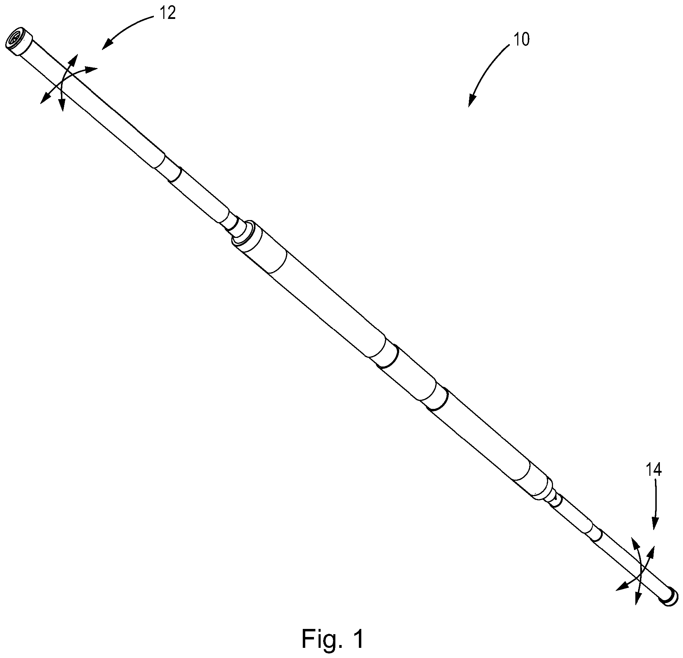

Fig. 1 zeigt ein endoskopisches Instrument 10 in einer perspektivischen Darstellung. Die Doppelpfeile am proximalen Ende 12 und am distalen Ende 14 symbolisieren, dass mittels einer Verlagerung am proximalen Ende 12 eine Verlagerung am distalen Ende 14 bewirkt werden kann.

Es sei darauf hingewiesen, dass die bisher und die nachfolgend verwendeten Bezugszeichen für alle Figuren und alle Ausführungsformen gelten, auch wenn die Bezugszeichen nicht in allen Figuren gezeigt sind und auch nicht in allen Figuren auf alle Bezugszeichen Bezug genommen wird.

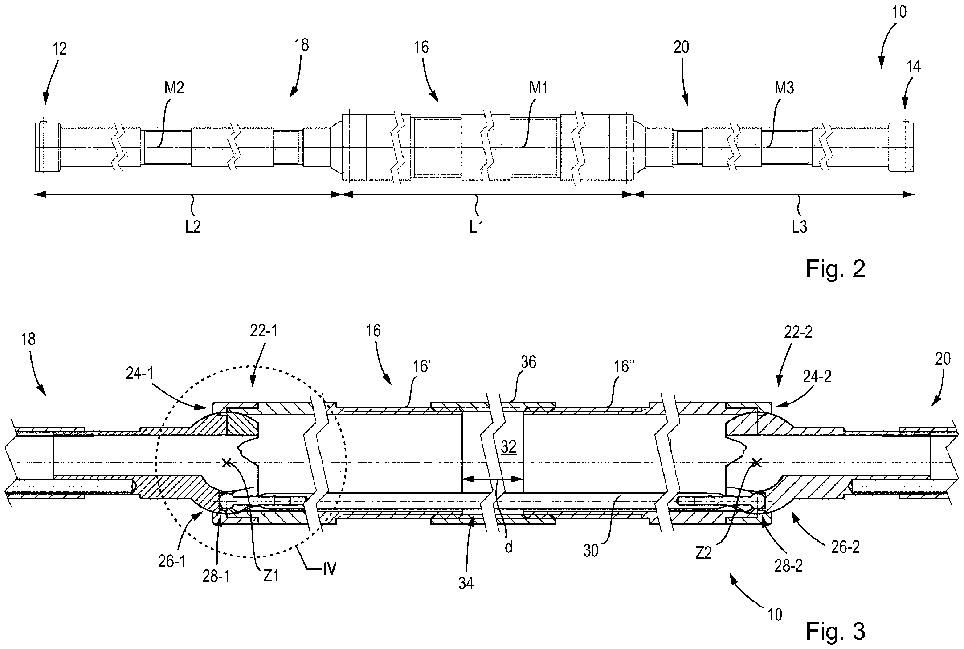

Fig. 2 zeigt das endoskopische Instrument 10 gemäß Fig. 1 in einer Seitenansicht. Das Instrument 10 weist einen ersten Abschnitt 16 mit einer ersten Längserstreckung L1 und einer Mittelachse M1, einen zweiten Abschnitt 18 mit einer zweiten Längserstreckung L2 und einer zweiten Mittelachse M2 und einen dritten Abschnitt 20 mit einer dritten Längserstreckung L3 und einer dritten Mittelachse M3 auf.

Fig. 3 zeigt das endoskopische Instrument 10 gemäß Fig. 2 in einer Schnittdarstellung, wobei der zweite Abschnitt 18 und der dritte Abschnitt 20 nur teilweise dargestellt sind. Das Instrument 10 weist ein erstes Hauptgelenk 22-1 auf, das ein erstes Hauptgelenkteil 24-1, ein weiteres erstes Hauptgelenkteil 26-1 und ein erstes Untergelenk 28-1 aufweist. Das Instrument 10 weist außerdem ein zweites Hauptgelenk 22-2 auf, das ein zweites Hauptgelenkteil 24-2, ein weiteres zweites Hauptgelenkteil 26-2 und ein zweites Untergelenk 28-2 aufweist.

Das Instrument 10 weist einen starren Hauptdruckstab 30 auf, der sich entlang der ersten Längserstreckung L1 und parallel zur ersten Mittelachse M1 erstreckt. Der erste Abschnitt 16 und der zweite Abschnitt 18 sind mittels des ersten Hauptgelenks 22-1 abwinkelbar miteinander verbunden. Der erste Abschnitt 16 und der dritte Abschnitt 20 sind mittels des zweiten Hauptgelenks 22-2 abwinkelbar miteinander verbunden.

Der Hauptdruckstab 30 ist mit dem ersten Untergelenk 28-1 und dem zweiten Untergelenk 28-2 gekoppelt, so dass eine Verlagerung des ersten Hauptgelenks 22-1 durch Abwinkelung des zweiten Abschnitts 18 relativ zum ersten Abschnitt 16 eine Kraft über den Hauptdruckstab 30 zum zweiten Hauptgelenk 22-2 überträgt und dort eine Abwinkelung des dritten Abschnitts 20 relativ zum ersten Abschnitt 16 bewirkt.

Bei der gezeigten Ausführungsform weisen das weitere erste Hauptgelenkteil 26-1 und das weitere zweite Hauptgelenkteil 26-2 während der Herstellung jeweils zwei Teile auf, deren Trennlinie jeweils zumindest ungefähr senkrecht zur ersten Mittelachse M1 und durch den ersten Mittelpunkt Z1 bzw. den zweiten Mittelpunkt Z2 verläuft. Die Enden des Hauptdruckstabs 30 weisen während der Herstellung auch jeweils zwei Teile auf. So kann der Teil mit der Gelenkkugel zwischen die Teile des weiteren ersten Hauptgelenkteils 26-1 bzw. des weiteren zweiten Hauptgelenkteils 26-2 eingelegt werden, die Teile zusammengefügt werden und der Teil mit der Gelenkkugel mit dem Mittelteil des Hauptdruckstabs 30 verbunden werden.

In der Schnittdarstellung ist zu erkennen, dass der erste Abschnitt 16 einen ersten Hohlraum 32 aufweist, der sich entlang der ersten Längserstreckung L1 erstreckt. Außerdem ist bei dieser Ausführungsform der ersten Abschnitt 16 an einer Stelle 34 entlang der ersten Längserstreckung L1 in einen ersten Abschnittsteil 16' und einen zweiten Abschnittsteil 16" geteilt. Der erste Abschnittsteil 16' und der zweite Abschnittsteil 16" sind mit einem Verbindungselement 36 derart miteinander verbunden, dass ein Abstand d zwischen dem ersten und dem zweiten Abschnittsteil 16', 16" einstellbar ist. Es ist schematisch dargestellt, dass das Verbindungselement 36 hier als Hülse mit einem Rechts-Links-Gewinde ausgebildet ist, das mit entsprechenden Gewinden an dem ersten und zweiten Abschnittsteil 16', 16" zusammenwirkt.

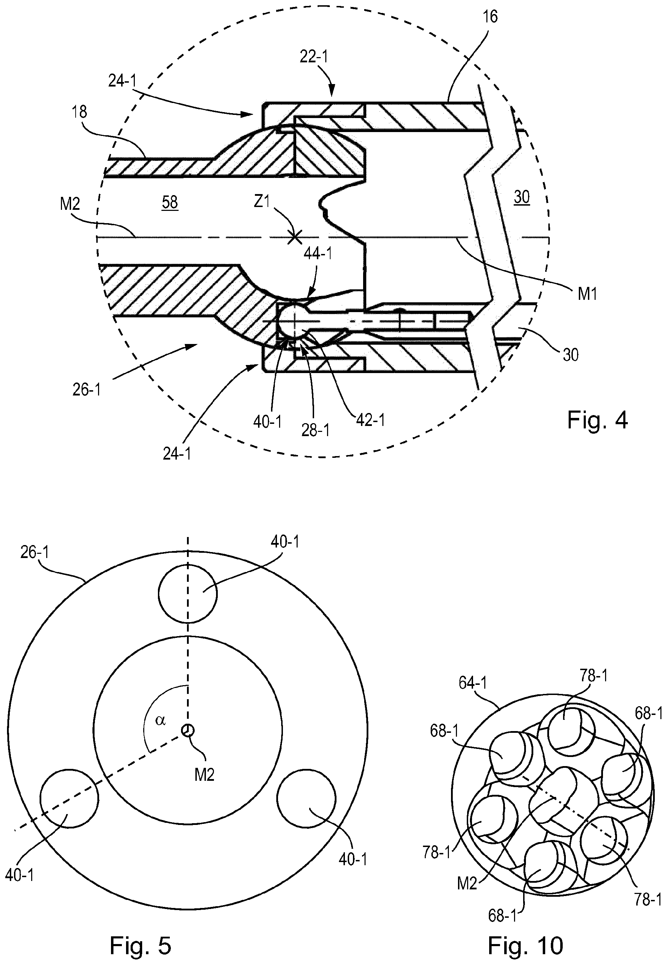

Fig. 4 zeigt eine Ausschnittsvergrößerung aus Fig. 3. Anhand dieser Figur soll das erste Hauptgelenk 22-1 erläutert werden. Die Erläuterungen zum Aufbau des ersten Hauptgelenks 22-1 gelten in entsprechender Weise auch für das zweite Hauptgelenk 22-2.

Wie bereits erläutert weist das erste Hauptgelenk 22-1 ein erstes Hauptgelenkteil 24-1, ein weiteres erstes Hauptgelenkteil 26-1 und ein erstes Untergelenk 28-1 auf. Das erste Hauptgelenkteil 24-1 wie auch das zweite Hauptgelenkteil 24-2 sind am ersten Abschnitt 16 angeordnet. Das weitere erste Hauptgelenkteil 26-1 ist am zweiten Abschnitt 18 angeordnet. In entsprechender Weise, hier nicht gezeigt, ist das weitere zweite Hauptgelenkteil 26-2 am dritten Abschnitt 20 angeordnet.

Das erste Hauptgelenkteil 24-1 ist zumindest als ein Abschnitt einer Gelenkpfanne ausgebildet. Das weitere erste Hauptgelenkteil 26-1 ist zumindest als Abschnitt einer Gelenkkugel ausgebildet. Diese Ausgestaltung ermöglicht eine Abwinkelung des zweiten Abschnitts 18 relativ zum ersten Abschnitt 16 in allen Richtungen. Das weitere erste Hauptgelenkteil 26-1 kann sich aber nicht entlang der ersten Längserstreckung L1 verlagern. Mit anderen Worten, ein erster Mittelpunkt Z1 des ersten Hauptgelenks 22-1 bleibt bei Verlagerungen des zweiten Abschnitts 18 stationär, auch entlang der ersten Längsrichtung L1.

Das erste Untergelenk 28-1 weist ein erstes Untergelenkteil 40-1 und ein weiteres erstes Untergelenkteil 42-1 auf. Bei der hier gezeigten Ausführungsform ist das erste Untergelenkteil 40-1 am weiteren ersten Hauptgelenkteil 26-1 ausgebildet. Das weitere erste Untergelenkteil 42-1 ist am Hauptdruckstab 30 ausgebildet.

Bei dieser Ausführungsform ist das erste Untergelenkteil 40-1 zumindest als ein Abschnitt einer Gelenkpfanne ausgebildet und ist das weitere erste Untergelenkteil 42-1 zumindest als ein Abschnitt einer Gelenkkugel ausgebildet. Das erste Untergelenkteil 40-1 ist mit einem Hinterschnitt 44-1 ausgebildet, so dass der Hauptdruckstab 30 sowohl einen positiven Druck als auch einen negativen Druck auf das weitere erste Hauptgelenkteil 26-1 ausüben kann.

Bei der hier gezeigten Ausführungsform sind am ersten Hauptgelenk 22-1 drei erste Untergelenke 28-1 angeordnet, die wie das in Fig. 4 gezeigte erste Untergelenk 28-1 ausgebildet sind und um die zweite Mittelachse M2 herum an verschiedenen Stellen des weiteren ersten Hauptgelenks 26-1 angeordnet sind.

Fig. 5 verdeutlicht die hier gewählte Anordnung, bei der die ersten Untergelenke 28-1, hier sind nur die ersten Untergelenkteile 40-1 schematisch dargestellt, in einem Abstand α von 120° relativ zueinander angeordnet sind, und zwar bezogen auf die zweite Mittelachse M2 oder auf die erste Mittelachse M1.

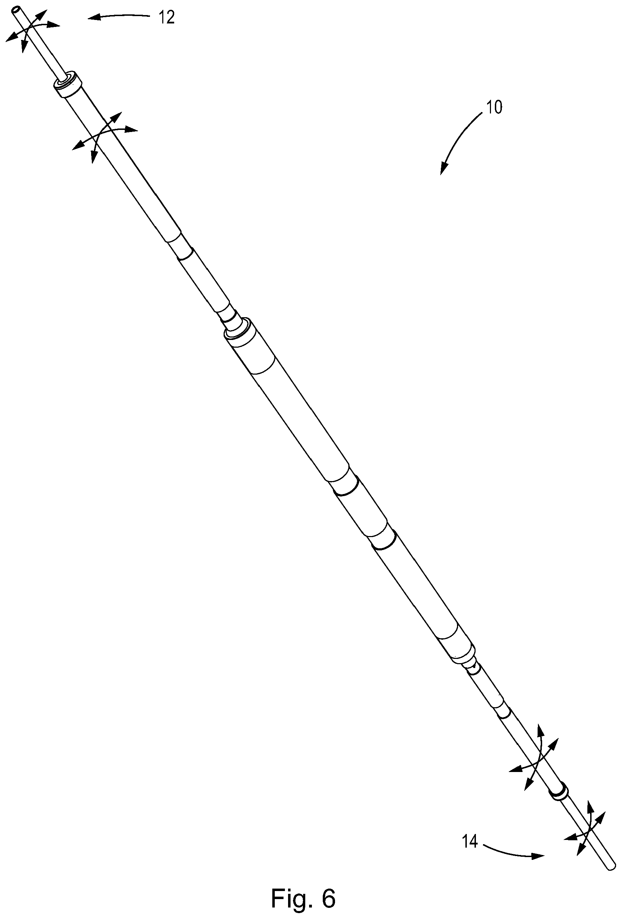

Fig. 6 zeigt eine zweite Ausführungsform eines endoskopischen Instruments 10 mit einem proximalen Ende 12 und einem distalen Ende 14.

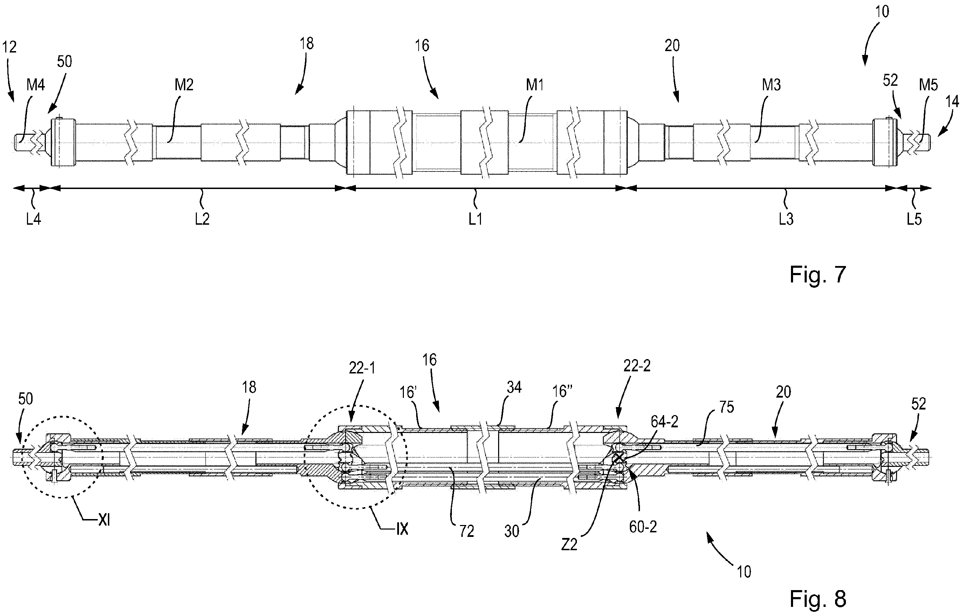

Fig. 7 zeigt das endoskopische Instrument 10 gemäß Fig. 6 in einer Seitenansicht. Es gelten alle Erläuterungen, die im Zusammenhang mit Fig. 2 gemacht wurden. Zusätzlich weist das Instrument 10 einen vierten Abschnitt 50 mit einer vierten Längserstreckung L4 und einer vierten Mittelachse M4 und einen fünften Abschnitt 52 mit einer fünften Längserstreckung L5 und einer fünften Mittelachse M5 auf.

Fig. 8 zeigt das endoskopische Instrument 10 gemäß Fig. 7 in einer Schnittdarstellung. Es gelten alle Ausführungen wie zur Fig. 3. Die Unterschiede zwischen der hier gezeigten zweiten Ausführungsform und der ersten Ausführungsform werden nachfolgend erläutert.

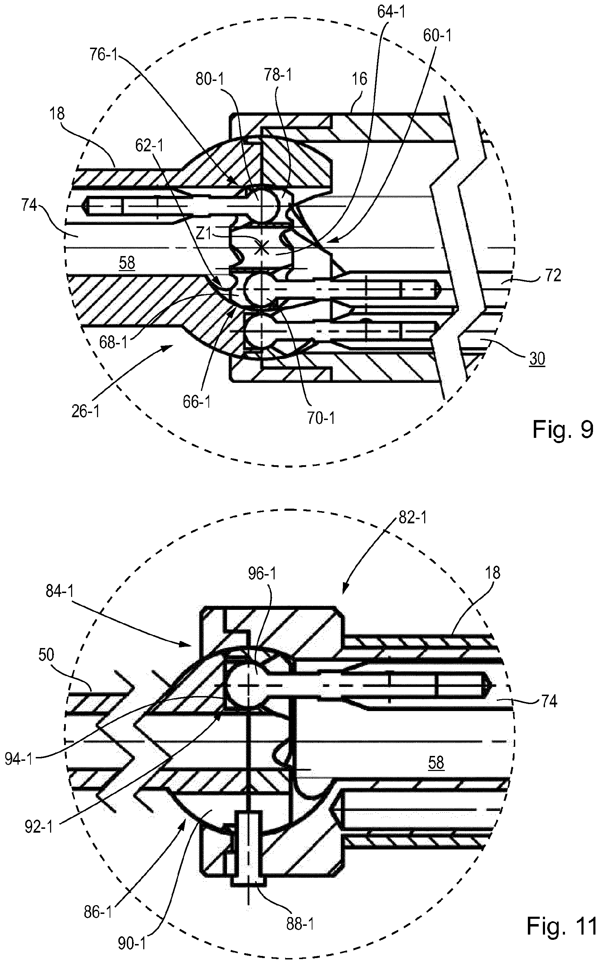

Fig. 9 zeigt eine Ausschnittsvergrößerung aus Fig. 8. Es gelten alle zur Fig. 4 gemachten Erläuterungen. Das weitere erste Hauptgelenkteil 26-1 weist ein erstes Innengelenk 60-1 auf, das im Inneren des weiteren ersten Hauptgelenkteils 26-1 angeordnet ist und relativ zum weiteren ersten Hauptgelenkteil 26-1 verschwenkbar angeordnet ist. Der zweite Abschnitt 18 weist entlang der zweiten Längserstreckung L2 einen zweiten Hohlraum 58 auf.

Das erste Innengelenk 60-1 weist ein erstes Innengelenkteil 62-1 und ein weiteres erstes Innengelenkteil 64-1 auf. Das erste Innengelenkteil 62-1 ist zumindest als ein Abschnitt einer Gelenkpfanne ausgebildet. Das weitere erste Innengelenkteil 64-1 ist zumindest als Abschnitt einer Gelenkkugel ausgebildet. Das erste Innengelenkteil 62-1 ist an dem weiteren ersten Hauptgelenkteil 26-1 ausgebildet. Mit anderen Worten ist die innere Oberfläche des weiteren ersten Hauptgelenkteils 26-1 so ausgebildet, dass sich das weitere erste Innengelenkteil 64-1 in dem weiteren ersten Hauptgelenkteil 26-1 wie in einer Gelenkpfanne verlagern kann.

Das weitere erste Innengelenkteil 64-1 weist ein erstes Primärgelenk 66-1 mit einem ersten Primärgelenkteil 68-1 und einem weiteren ersten Primärgelenkteil 70-1 auf. Das erste Primärgelenkteil 68-1 ist an dem weiteren ersten Innengelenkteil 64-1 ausgebildet, hier in der Form eines Abschnitts einer Gelenkpfanne, der aufgrund der Schnittdarstellung nur teilweise zu erkennen ist. Das endoskopische Instrument weist ferner einen starren Nebendruckstab 72 auf, der sich entlang der ersten Längserstreckung L1 erstreckt, wobei das weitere erste Primärgelenkteil 70-1 am Nebendruckstab 72 ausgebildet ist. Das erste Primärgelenk 66-1 ist so ausgebildet, dass über den Nebendruckstab 72 sowohl ein positiver Druck als auch ein negativer Druck auf das weitere erste Innengelenkteil 64-1 ausgeübt werden können.

Das Instrument 10 weist ferner einen ersten starren Seitendruckstab 74 auf, der sich entlang der zweiten Längserstreckung L2 erstreckt. Das weitere erste Innengelenkteil 64-1 weist außerdem ein erstes Sekundärgelenk 76-1 mit einem ersten Sekundärgelenkteil 78-1 und einem weiteren ersten Sekundärgelenkteil 80-1 auf. Das erste Sekundärgelenkteil 78-1 ist an dem weiteren ersten Innengelenkteil 64-1 ausgebildet, und das weitere erste Sekundärgelenkteil 80-1 ist am ersten Seitendruckstab 74 ausgebildet.

Alle Erläuterungen zur Fig. 9 gelten in entsprechender Weise auch für das zweite Innengelenk 60-2.

Zur Verdeutlichung der Funktionsweise sollen zwei beispielhafte Anwendungsmöglichkeiten anhand der Figuren 8 und 9 erläutert werden. Zum einen kann der erste Seitendruckstab 74, bezogen auf die Orientierung in der Zeichnung, nach rechts verlagert werden. Dadurch rotiert das weitere erste Innengelenkteil 64-1 im Uhrzeigersinn um den ersten Mittelpunkt Z1. Dies führt dazu, dass das erste Primärgelenk 66-1 ebenfalls um den ersten Mittelpunkt Z1 verlagert wird und den Nebendruckstab 72 nach links verlagert. Der Nebendruckstab 72, der auch mit dem zweiten weiteren Innengelenkteil 64-2 gekoppelt ist, verlagert das weitere zweite Innengelenkteil 64-2 im Uhrzeigersinn um den zweiten Mittelpunkt Z2. Dadurch wird der zweite Seitendruckstab 75 nach rechts verlagert.

Im zweiten Fall wird der zweite Abschnitt 18 im Uhrzeigersinn um den ersten Mittelpunkt Z1 abgewinkelt. Diese Abwinklung zieht den Hauptdruckstab 30 nach links. Da der Hauptdruckstab 30 mit dem weiteren zweiten Hauptgelenkteil 26-2 gekoppelt ist, wird der dritte Abschnitt 20 um den zweiten Mittelpunkt Z2 im Uhrzeigersinn verlagert.

Fig. 10 zeigt das weitere erste Innengelenkteil 64-1 in einer perspektivischen Darstellung. Es ist zu erkennen, dass die ersten Primärgelenkteile 68-1 und die ersten Sekundärgelenkteile 78-1 alternierend in einer Art Ring um die zweite Mittelachse M2 angeordnet sind. Bei der Betätigung des Instruments 10 gehören funktional jeweils ein erstes Primärgelenkteil 68-1 und ein diametral gegenüberliegendes erstes Sekundärgelenkteil 78-1 zusammen. Die vorgeschlagene Ausgestaltung des weiteren ersten Innengelenkteils 64-1 wird als Gelenkpfannenkranz bezeichnet.

Fig. 11 zeigt eine weitere Ausschnittsvergrößerung aus Fig. 8. Es ist ein erstes Seitengelenk 82-1 gezeigt, das ein erstes Seitengelenkteil 84-1 und ein weiteres erstes Seitengelenkteil 86-1 aufweist. Ein erster Führungsstift 88-1 greift in eine erste Gelenknut 90-1 ein und verhindert eine Rotation des vierten Abschnitts 50 um die vierte Mittelachse M4.

Das weitere erste Seitengelenkteil 86-1 weist außerdem ein erstes Seitenuntergelenk 92-1 auf, das aus einem ersten Seitenuntergelenkteil 94-1 und einem weiteren ersten Seitenuntergelenkteil 96-1 gebildet ist. Das erste Seitenuntergelenkteil 94-1 ist zumindest als ein Abschnitt einer Gelenkpfanne ausgebildet, und das weitere erste Seitenuntergelenkteil 96-1 ist zumindest als Abschnitt einer Gelenkkugel ausgebildet. Das weitere erste Seitenuntergelenkteil 96-1 ist an dem ersten Seitendruckstab 74 ausgebildet. Daher führt eine Abwinklung des vierten Abschnitts 50 zu einer Verlagerung des ersten Seitendruckstabs 74.

Dieser Aufbau ermöglicht im Ergebnis unter anderem, dass durch eine Abwinklung des vierten Abschnitts 50 eine Abwinklung des fünften Abschnitts 52 bewirkt werden kann und durch eine Abwinklung des zweiten Abschnitts 18 eine Abwinklung des dritten Abschnitts 20 bewirkt werden kann. Da die genannten Druckstäbe starr sind, können positive und negative Druckkräfte besonders zuverlässig übertragen werden.

## Patentansprüche

1. Endoskopisches Instrument (10) mit
einem ersten Abschnitt (16), der eine erste Längserstreckung (L1) aufweist, einem zweiten Abschnitt (18) und einem dritten Abschnitt (20),
einem ersten Hauptgelenk (22-1), das ein erstes Hauptgelenkteil (24-1), ein weiteres erstes Hauptgelenkteil (26-1) und ein erstes Untergelenk (28-1) aufweist,
einem zweiten Hauptgelenk (22-2), das ein zweites Hauptgelenkteil (24-2), ein weiteres zweites Hauptgelenkteil (26-2) und ein zweites Untergelenk (28-2) aufweist, und
einem starren Hauptdruckstab (30), der sich entlang der ersten Längserstreckung (L1) erstreckt,
wobei der erste Abschnitt (16) und der zweite Abschnitt (18) mittels des ersten Hauptgelenks (22-1) abwinkelbar miteinander verbunden sind, und
wobei der erste Abschnitt (16) und der dritte Abschnitt (20) mittels des zweiten Hauptgelenks (22-2) abwinkelbar miteinander verbunden sind,
wobei der Hauptdruckstab (30) mit dem ersten Untergelenk (28-1) und dem zweiten Untergelenk (28-2) gekoppelt ist, so dass eine Verlagerung des ersten Hauptgelenks (22-1) durch Abwinkelung des zweiten Abschnitts (18) relativ zum ersten Abschnitt (16) eine Kraft über den Hauptdruckstab (30) zum zweiten Hauptgelenk (22-2) überträgt und dort eine Abwinkelung des dritten Abschnitts (20) relativ zum ersten Abschnitt (16) bewirkt, und
wobei das weitere erste Hauptgelenkteil (26-1) ein erstes Innengelenkteil (62-1) und ein weiteres erstes Innengelenkteil (64-1) aufweist, wobei das erste Innengelenkteil (62-1) zumindest als ein Abschnitt einer Gelenkpfanne an dem weiteren ersten Hauptgelenkteil (26-1) ausgebildet ist, und wobei das weitere erste Innengelenkteil (64-1) zumindest als ein Abschnitt einer Gelenkkugel ausgebildet ist und im Inneren des weiteren ersten Hauptgelenkteils (26-1) angeordnet ist, wobei das weitere erste Innengelenkteil (64-1) relativ zum weiteren ersten Hauptgelenkteil (26-1) und zum ersten Untergelenk (28-1) verschwenkbar angeordnet ist, wobei ein Verschwenken des weiteren ersten Innengelenkteils (64-1) ohne Verlagerung des ersten Untergelenks (28-1) erfolgen kann und wobei der zweite Abschnitt (18) eine zweite Längserstreckung (L2) und einen zweiten Hohlraum (58) entlang der zweiten Längserstreckung (L2) aufweist.

2. Endoskopisches Instrument nach Anspruch 1, wobei das erste Hauptgelenkteil (24-1) und das zweite Hauptgelenkteil (24-2) am ersten Abschnitt (16) angeordnet sind, das weitere erste Hauptgelenkteil (26-1) am zweiten Abschnitt (18) angeordnet ist und das weitere zweite Hauptgelenkteil (26-2) am dritten Abschnitt (20) angeordnet ist.

3. Endoskopisches Instrument nach einem der vorhergehenden Ansprüche, wobei das erste Hauptgelenkteil (24-1) zumindest als ein Abschnitt einer Gelenkpfanne ausgebildet und das weitere erste Hauptgelenkteil (26-1) zumindest als Abschnitt einer Gelenkkugel ausgebildet ist.

4. Endoskopisches Instrument nach einem der vorhergehenden Ansprüche, wobei das erste Untergelenk (28-1) ein erstes Untergelenkteil (40-1) und ein weiteres erstes Untergelenkteil (42-1) aufweist, wobei das erste Untergelenkteil (40-1) am weiteren ersten Hauptgelenkteil (26-1) ausgebildet ist und wobei das weitere erste Untergelenkteil (42-1) am Hauptdruckstab (30) ausgebildet ist.

5. Endoskopisches Instrument nach Anspruch 4, wobei das erste Untergelenkteil (40-1) zumindest als ein Abschnitt einer Gelenkpfanne ausgebildet und das weitere erste Untergelenkteil (42-1) zumindest als Abschnitt einer Gelenkkugel ausgebildet ist.

6. Endoskopisches Instrument nach einem der vorhergehenden Ansprüche, wobei am ersten Hauptgelenk (22-1) drei erste Untergelenke (28-1) angeordnet sind.

7. Endoskopisches Instrument nach Anspruch 6, wobei die ersten Untergelenke (28-1) in einem Abstand α von zwischen 45° und 120° relativ zueinander angeordnet sind, und zwar bezogen auf eine erste Mittelachse (M1) des ersten Abschnitts (16), die durch einen ersten Mittelpunkt (Z1) des ersten Hauptgelenks (22-1) und durch einen zweiten Mittelpunkt (Z2) des zweiten Hauptgelenks verläuft.

8. Endoskopisches Instrument nach einem der vorhergehenden Ansprüche, wobei das weitere erste Innengelenkteil (64-1) ein erstes Primärgelenk (66-1) mit einem ersten Primärgelenkteil (68-1) und einem weiteren ersten Primärgelenkteil (70-1) aufweist, wobei das erste Primärgelenkteil (68-1) an dem weiteren ersten Innengelenkteil (64-1) ausgebildet ist.

9. Endoskopisches Instrument nach Anspruch 8, ferner mit einem starren Nebendruckstab (72), der sich entlang der ersten Längserstreckung (L1) erstreckt, wobei das weitere erste Primärgelenkteil (70-1) am Nebendruckstab (72) ausgebildet ist.

10. Endoskopisches Instrument nach einem der Ansprüche 8 bis 9, ferner mit einem ersten starren Seitendruckstab (74), der sich entlang der zweiten Längserstreckung (L2) erstreckt, wobei das weitere erste Innengelenkteil (64-1) ein erstes Sekundärgelenk (76-1) mit einem ersten Sekundärgelenkteil (78-1) und einem weiteren ersten Sekundärgelenkteil (80-1) aufweist, wobei das erste Sekundärgelenkteil (78-1) an dem weiteren ersten Innengelenkteil (64-1) ausgebildet ist und das weitere erste Sekundärgelenkteil (80-1) am ersten Seitendruckstab (74) ausgebildet ist.

11. Endoskopisches Instrument nach Anspruch 10, wobei das weitere erste Innengelenkteil (64-1) drei erste Primärgelenke und drei erste Sekundärgelenke aufweist.

12. Endoskopisches Instrument nach einem der vorhergehenden Ansprüche, wobei der erste Abschnitt (16) an einer Stelle (34) entlang der ersten Längserstreckung (L1) in einen ersten Abschnittsteil (16') und einen zweiten Abschnittsteil (16") geteilt ist, und der erste Abschnittsteil (16') und der zweite Abschnittsteil (16") mit einem Verbindungselement (36) derart verbunden sind, dass ein Abstand (d) zwischen dem ersten und dem zweiten Abschnittsteil (16', 16") einstellbar ist.

## Claims

1. An endoscopic instrument (10) with
a first portion (16) having a first longitudinal extension (L1),
a second portion (18) and a third portion (20),
a first main joint (22-1) comprising a first main joint part (24-1), a further first main joint part (26-1) and a first sub-joint (28-1),
a second main joint (22-2) comprising a second main joint part (24-2), a further second main joint part (26-2) and a second sub-joint (28-2), and
a rigid main pressure rod (30) extending along said first longitudinal extension (L1), the first portion (16) and the second portion (18) being connected to each other in a deflectable manner by means of the first main joint (22-1), and
wherein the first portion (16) and the third portion (20) are connected to each other in a deflectable manner by means of the second main joint (22-2),
the main pressure rod (30) being coupled to the first sub-joint (28-1) and the second sub-joint (28-2) such that displacement of the first main joint (22-1) by deflecting the second portion (18) relative to the first portion (16) transmits a force via the main pressure rod (30) to the second main joint (22-2) and causes deflection of the third portion (20) relative to the first portion (16), and
the further first main joint part (26-1) comprising a first inner joint part (62-1) and a further first inner joint part (64-1), wherein the first inner joint part (62-1) is formed at least as a part of a socket on the further first main joint part (26-1), and wherein the further first inner joint part (64-1) is formed at least as a part of a ball joint and is disposed within the further first main joint part (26-1), wherein the further first inner joint part (64-1) is pivotally disposed relative to the further first main joint part (26-1) and to the first sub-joint (28-1), wherein pivoting of the further first inner joint part (64-1) can occur without displacement of the first sub-joint (28-1), and wherein the second portion (18) has a second longitudinal extension (L2) and a second cavity (58) along the second longitudinal extension (L2).

2. The endoscopic instrument according to claim 1, wherein the first main joint part (24-1) and the second main joint part (24-2) are disposed on the first portion (16), the further first main joint part (26-1) is disposed on the second portion (18), and the further second main joint part (26-2) is disposed on the third portion (20).

3. The endoscopic instrument according to one of the preceding claims, wherein the first main joint part (24-1) is formed at least as a part of an articular socket and the further first main joint part (26-1) is formed at least as a part of a joint ball.

4. The endoscopic instrument according to one of the preceding claims, wherein the first sub-joint (28-1) comprises a first sub-joint part (40-1) and a further first sub-joint part (42-1), wherein the first sub-joint part (40-1) is formed on the further first main joint part (26-1) and wherein the further first sub-joint part (42-1) is formed on the main pressure rod (30).

5. The endoscopic instrument according to claim 4, wherein the first sub-joint part (40-1) is formed at least as a part of an articular socket and the further first sub-joint part (42-1) is formed at least as a part of a joint ball.

6. The endoscopic instrument according to one of the preceding claims, wherein three first sub-joints (28-1) are arranged at the first main joint (22-1).

7. The endoscopic instrument according to claim 6, wherein the first sub-joints (28-1) are disposed at a distance of α between 45° and 120° relative to each other with respect to a first center axis (M1) of the first portion (16) passing through a first center (Z1) of the first main joint (22-1) and through a second center (Z2) of the second main joint.

8. The endoscopic instrument according to one of the preceding claims, the further first inner joint part (64-1) comprising a first primary joint (66-1) having a first primary joint part (68-1) and a further first primary joint part (70-1), the first primary joint part (68-1) being formed on the further first inner joint part (64-1).

9. The endoscopic instrument according to claim 8 further comprising a rigid secondary pressure rod (72) extending along the first longitudinal extension (L1), the further first primary joint part (70-1) being formed on the secondary pressure rod (72).

10. The endoscopic instrument according to any of claims 8 to 9, further comprising a first rigid lateral pressure rod (74) extending along the second longitudinal extension (L2), the further first inner joint part (64-1) comprising a first secondary joint (76-1) having a first secondary joint part (78-1) and a further first secondary joint part (80-1), the first secondary joint part (78-1) being formed on the further first inner joint part (64-1) and the further first secondary joint part (80-1) being formed on the first lateral pressure rod (74).

11. The endoscopic instrument according to claim 10, wherein the further first inner joint part (64-1) comprises three first primary joints and three first secondary joints.

12. The endoscopic instrument according to one of the preceding claims, wherein the first section (16) is divided at a location (34) along the first longitudinal extent (L1) into a first section part (16') and a second section part (16"), and the first section part (16') and the second section part (16") are connected to a connecting element (36) such that a distance (d) between the first and the second section part (16', 16") is adjustable.

## Revendications

1. Instrument endoscopique (10), comprenant
une première portion (16) qui présente une première étendue longitudinale (L1), une deuxième portion (18) et une troisième portion (20),
une première articulation principale (22-1), qui présente une première partie d'articulation principale (24-1), une première partie d'articulation principale supplémentaire (26-1) et une première sous-articulation (28-1),
une deuxième articulation principale (22-2) qui présente une deuxième partie d'articulation principale (24-2), une deuxième partie d'articulation principale supplémentaire (26-2) et une deuxième sous-articulation (28-2), et
une barre de pression principale rigide (30) qui s'étend le long de la première étendue longitudinale (L1),
la première portion (16) et la deuxième portion (18) étant reliées l'une à l'autre de manière à pouvoir être coudées au moyen de la première articulation principale (22-1), et
la première portion (16) et la troisième portion (20) étant reliées l'une à l'autre de manière à pouvoir être coudées au moyen de la deuxième articulation principale (22-2),
la barre de pression principale (30) étant accouplée à la première sous-articulation (28-1) et à la deuxième sous-artiuclation (28-2) de telle sorte qu'un déplacement de la première articulation principale (22-1) par coudage de la deuxième portion (18) par rapport à la première portion (16) transfère une force par le biais de la barre de pression principale (30) à la deuxième articulation principale (22-2) et provoque ainsi un coudage de la troisième portion (20) par rapport à la première portion (16), et
la première partie d'articulation principale supplémentaire (26-1) présentant une première partie d'articulation interne (62-1) et une première partie d'articulation interne supplémentaire (64-1), la première partie d'articulation interne (62-1) étant réalisée au moins sous la forme d'une portion d'une cavité articulaire au niveau de la première partie d'articulation principale supplémentaire (26-1), et la première partie d'articulation interne supplémentaire (64-1) étant réalisée au moins sous la forme d'une portion d'une sphère d'articulation et étant disposée à l'intérieur de la première partie d'articulation principale supplémentaire (26-1), la première partie d'articulation interne supplémentaire (64-1) étant disposée de manière à pouvoir pivoter par rapport à la première partie d'articulation principale supplémentaire (26-1) et à la première sous-articulation (28-1), un pivotement de la première partie d'articulation interne supplémentaire (64-1) pouvant s'effectuer sans déplacement de la première sous-articulation (28-1) et la deuxième portion (18) présentant une deuxième étendue longitudinale (L2) et une deuxième cavité (58) le long de la deuxième étendue longitudinale (L2).

2. Instrument endoscopique selon la revendication 1, dans lequel la première partie d'articulation principale (24-1) et la deuxième partie d'articulation principale (24-2) sont disposées au niveau de la première portion (16), la première partie d'articulation principale supplémentaire (26-1) est disposée au niveau de la deuxième portion (18) et la deuxième partie d'articulation principale supplémentaire (26-2) est disposée au niveau de la troisième portion (20).

3. Instrument endoscopique selon l'une quelconque des revendications précédentes, dans lequel la première partie d'articulation principale (24-1) est réalisée au moins sous la forme d'une portion d'une cavité articulaire et la première partie d'articulation principale supplémentaire (26-1) est réalisée au moins sous forme de portion d'une sphère d'articulation.

4. Instrument endoscopique selon l'une quelconque des revendications précédentes, dans lequel la première sous-articulation (28-1) présente une première partie sous-articulation (40-1) et une première partie sous-articulation supplémentaire (42-1), la première partie sous-articulation (40-1) étant réalisée au niveau de la première partie d'articulation principale supplémentaire (26-1) et la première partie sous-articulation supplémentaire (42-1) étant réalisée au niveau de la barre de pression principale (30).

5. Instrument endoscopique selon la revendication 4, dans lequel la première partie d'articulation secondaire (40-1) est réalisée au moins sous la forme d'une portion d'une cavité articulaire et la première partie de sous-articulation supplémentaire (42-1) est réalisée au moins sous forme de portion d'une sphère d'articulation.

6. Instrument endoscopique selon l'une quelconque des revendications précédentes, dans lequel trois premières sous-articulations (28-1) sont disposées au niveau de la première articulation principale (22-1).

7. Instrument endoscopique selon la revendication 6, dans lequel les premières sous-articulations (28-1) sont disposées à une distance α les unes des autres comprises entre 45° et 120°, et ce par rapport à un premier axe médian (M1) de la première portion (16) qui s'étend à travers un premier centre (Z1) de la première articulation principale (22-1) et à travers un deuxième centre (Z2) de la deuxième articulation principale.

8. Instrument endoscopique selon l'une quelconque des revendications précédentes, dans lequel la première partie d'articulation interne supplémentaire (64-1) présente une première articulation primaire (66-1) avec une première partie d'articulation primaire (68-1) et une première partie d'articulation primaire supplémentaire (70-1), la première partie d'articulation primaire (68-1) étant réalisée au niveau de la première partie d'articulation interne supplémentaire (64-1).

9. Instrument endoscopique selon la revendication 8, comprenant en outre une barre de pression auxiliaire rigide (72) qui s'étend le long de la première étendue longitudinale (L1), la première partie d'articulation primaire supplémentaire (70-1) étant réalisée au niveau de la barre de pression auxiliaire (72).

10. Instrument endoscopique selon l'une quelconque des revendications 8 et 9, comprenant en outre une première barre de pression latérale rigide (74) qui s'étend le long de la deuxième étendue longitudinale (L2), la première partie d'articulation interne supplémentaire (64-1) présentant une première articulation secondaire (76-1) avec une première partie d'articulation secondaire (78-1) et une première partie d'articulation secondaire supplémentaire (80-1), la première partie d'articulation secondaire (78-1) étant réalisée au niveau de la première partie d'articulation interne supplémentaire (64-1) et la première partie d'articulation secondaire supplémentaire (80-1) étant réalisée au niveau de la première barre de pression latérale (74).

11. Instrument endoscopique selon la revendication 10 dans lequel la première partie d'articulation interne supplémentaire (64-1) présente trois premières articulations primaires et trois premières articulations secondaires.

12. Instrument endoscopique selon l'une quelconque des revendications précédentes, dans lequel la première portion (16) est divisée au niveau d'un endroit (34) le long de la première étendue longitudinale (L1) en une première partie de portion (16') et une deuxième partie de portion (16"), et la première partie de portion (16') et la deuxième partie de portion (16") sont connectées à un élément de connexion (36) de telle sorte qu'une distance (d) entre la première et la deuxième partie de portion (16', 16") puisse être ajustée.
